# EUROPEAN PATENT SPECIFICATION

(11) **EP 1 585 523 B1**
(45) Date of publication and mention of the grant of the patent: **01.10.2008**
(21) Application number: 03812071.3
(22) Date of filing: 17.12.2003
(51) Int. Cl.: A61K 31/52, A61P 27/16

(54) **USE OF PURINE DERIVATIVES FOR INDUCING DIFFERENTIATION OF SUPERNUMERARY HAIR CELLS AND DEITERS' CELLS IN THE DEVELOPING ORGAN OF CORTI FOR TREATING DEAFNESS**
VERWENDUNG VON PURINDERIVATEN ZUR INDUKTION DER DIFFERENZIERUNG ÜBERZÄHLIGER HAARZELLEN UND DEITER-ZELLEN IM ENTWICKELNDEN CORTI-ORGAN ZUR BEHANDLUNG VON TAUBHEIT
TECHNIQUE PERMETTANT D'INDUIRE LA DIFFERENTIATION DE CELLULES PILEUSES SURNUMERAIRES ET DE CELLULES DE DEITER DANS L'ORGANE DE CORTI EN DEVELOPPEMENT

(30) Priority: 17.12.2002 US 433767 P; 18.12.2002 US 433960 P
(43) Date of publication of application: 19.10.2005
(73) Proprietor: CENTRE NATIONAL DE LA RECHERCHE SCIENTIFIQUE (CNRS), 75794 Paris Cedex 16 (FR)
(72) Inventor: MEIJER, Laurent, F-29682 Roscoff (FR); KNOCKAERT, Marie, F-29680 Roscoff (FR); MALGRANGE, Brigitte, B-4053 Embourg (BE)
(74) Representative: Peaucelle, Chantal
(86) International application number: PCT/EP2003/015047
(87) International publication number: WO 2004/055154

(56) References cited:
- WO-A-97/20842
- WO-A-99/42088
- LÖWENHEIM H ET AL: "Gene disruption of p27(Kip1) allows cell proliferation in the postnatal and adult organ of corti." PROCEEDINGS OF THE NATIONAL ACADEMY OF SCIENCES OF THE UNITED STATES OF AMERICA. 30 MAR 1999, vol. 96, no. 7, 30 March 1999 (1999-03-30), pages 4084-4088, XP002294147 ISSN: 0027-8424
- CHEN PING ET AL: "p27Kip1 links cell proliferation to morphogenesis in the developing organ of Corti" DEVELOPMENT (CAMBRIDGE), vol. 126, no. 8, April 1999 (1999-04), pages 1581-1590, XP002294148 ISSN: 0950-1991
- KNOCKAERT M ET AL: "Pharmacological inhibitors of cyclin-dependent kinases" TRENDS IN PHARMACOLOGICAL SCIENCES, ELSEVIER, AMSTERDAM, NL, vol. 23, no. 9, 1 September 2002 (2002-09-01), pages 417-425, XP004381043 ISSN: 0165-6147
- LEGRAVEREND M ET AL: "Cyclin-dependent kinase inhibition by new C-2 alkynylated purine derivatives and molecular structure of a CDK2-inhibitor complex" JOURNAL OF MEDICINAL CHEMISTRY, AMERICAN CHEMICAL SOCIETY. WASHINGTON, US, vol. 43, no. 7, 2000, pages 1282-1292, XP002248272 ISSN: 0022-2623
- LEGRAVEREND M ET AL: "Synthesis of C2 alkynylated purines, a new family of potent inhibitors of cyclin-dependent kinases" BIOORGANIC & MEDICINAL CHEMISTRY LETTERS, OXFORD, GB, vol. 8, no. 7, 7 April 1998 (1998-04-07), pages 793-798, XP004136967 ISSN: 0960-894X

## Description

The invention relates to means for inducing differentiation of supernumerary hair cells and Deiters'cells in the developing organ of Corti and their uses for treating deafness.

The mammalian organ of Corti has a highly organized tissue patterning mode of intricate sensory and non-sensory cells. Molecular cues that drive this strict arrangement remain broadly unknown. In the embryonic day 19 organs of Corti, it was shown that roscovitine, a chemical inhibitor of cyclin-dependent kinases, significantly increased the number of hair cells (HCs) and corresponding supporting cells (SCs) by triggering differentiation of precursor cells without interacting with cell proliferation. The effect of roscovitine was mimicked by other CDK1, 2, 5, 7, inhibitors, but not by CDK4/6 and MAP kinase pathway antagonists. Immunohistochemical analysis indicated that roscovitine specific intracellular targets, CDK1, 2, 5, 7 and ERK1/2 were expressed in the organ of Corti and especially in Hensen's cells. Affinity chromatography studies showed a tight correlation between the protein levels of CDK1, 2 and 5 with the rate of roscovitine-induced supernumerary cells in the organ of Corti. In addition, it was demonstrated that basal kinase activity was higher and more roscovitine sensitive at the specific developmental stages allowing the emergence of supernumerary cells. The effects of roscovitine required the Math1 transcription factor, as shown by the lack of effect of roscovitine on organs of Corti derived from Math1-null mice. These results showed that cyclin-dependent kinases are involved in the normal development of the organ of Corti and that, at least in E19 embryos, inhibition of CDKs is sufficient to trigger the differentiation of HCs and SCs, presumably from the Hensen's cells progenitors. The effects of roscovitine may be due either to the inhibition of phosphorylation (CDK1/2/5 inhibition) or inhibition of synthesis (CDK7 inhibition) of factors repressing differentiation into the HC phenotype.

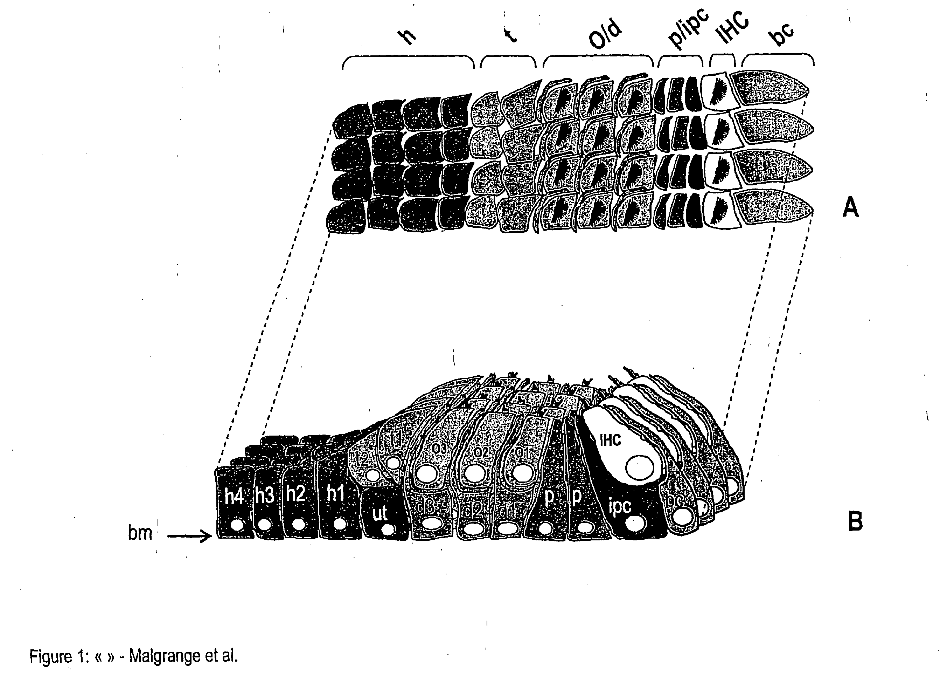

Development of the organ of Corti, the auditory sense organ of mammals, involves the differentiation of sensory hair cells (HC) (inner and outer) and non sensory supporting cells (SC) including Deiters' cells, pillar cells, inner phalangeal cells, tectal cells and Hensen's cells (Figure 1). Each of these cell types has a distinct morphology that contributes to the complex structural and functional properties of the organ of Corti. Production of new HCs occurs throughout life in the auditory and vestibular sensory receptor of fishes and amphibians and in the vestibular receptors of birds. In contrast, ongoing production of auditory HCs during post-embryonic life does not appear to occur in avian auditory sensory epithelium, although HCs lost after sound trauma- or ototoxin-induced death are replaced.
In mammals, embryonic HCs and SCs proliferation within the sensory epithelia culminates between embryonic days 13 (E13) and 15 (E15) while neonatal HC production never occurs under normal conditions.

In higher eucaryotes, the commitment to cellular differentiation is controlled by series of highly scheduled events, involving sequential signals that regulate decisions of cell cycle exit, cell survival and the onset of differentiation process. The choice to remain into or to withdraw from the cell cycle and to differentiate is made during the G1 phase of the cell cycle by the interaction of G1 cyclins, cyclin-dependent kinases (CDKs) and endogenous CDK inhibitors (CKI). Two classes of these endogenous CKIs have been identified: the INK4 family specifically inhibiting cyclin D-associated CDKs (CDK4 and CDK6), and the Cip/Kip family including p21, p27, and p57, inhibiting all types of CDK/cyclin complexes.
Cip/Kip proteins are thought to be critical terminal effectors of the signal transduction pathways that control cell differentiation. p27^{KIP1}, which specifically inhibits CDK2, 4 and 6, has been shown to be an essential mediator of oligodendrocyte terminal differentiation.
p27^{KIP1} expression is induced in the primordial organ of Corti between E12 and E14, correlating at this stage with cell division arrest in HCs' and SCs' progenitors. In wild-type animals, p27^{KIP1} expression is down-regulated during subsequent HC differentiation, but persists at high levels in differentiated SCs of the mature organ of Corti. In mice with a targeted deletion of the p27^{KIP1} gene, spontaneous supernumerary HCs and SCs are present in the postnatal organ of Corti.
Ectopic expression of CDK2, but not CDK4 or any of the cyclin, inhibits NGF-mediated neuronal differentiation of PC12 cells.
In addition, cell differentiation has been reported to correlate with the suppression or activation of kinase activity in several cell types including those of the neuronal, hematopoietic and myocytic lineages.
Taken together, it is clear that CDKs play a crucial ubiquitous role in the fine tuning of the balance between cell proliferation and differentiation, but the specific CDKs involved may vary between distinct cell phenotypes.

The inventors have investigated the function of cell cycle regulatory molecules in HC and SC differentiation of embryonic day 19 (E19) rat organs of Corti. The E19 rat organ of Corti is a well suited model of HC and SC differentiation since culturing these explants results in the spontaneous appearance of supernumerary HCs and corresponding SCs via differentiation of Hensen's cells in 10% of the explants regions.

The inventors have found that roscovitine, a selective inhibitor of the cyclin-dependent kinases CDK1, CDK2, CDK5, CDK7 and, to a lesser extent, of ERK1/ERK2, as well as the other kinase inhibitors, such as purine derivatives, indirubins, purvulanols, significantly increases the appearance of supernumerary HCs and corresponding SCs in E19 rat organ of Corti after 5 days in vitro (DIV). A rapid decline in the formation of supernumerary HCs and corresponding SCs in response to roscovitine is observed between E19 and P0. This lack of responsiveness to roscovitine is associated with the decline of the CDK1, 2 and 5 expression and CDK1/2 enzymatic activity. CDK1,2,5 and 7 are preferentially localized in Hensen's cells, the proposed progenitor cells from which HCs and SCs differentiate.

Altogether, these results show that pharmacological CDK inhibitors may have a direct inducing effect on the differentiation of HCs and corresponding SCs from Hensen's cells.

Deletion of the mouse basic helix-loop-helix (bHLH) transcription factor *Math1* causes failure of HCs generation in the cochlea. In addition, *Math1* has been shown to be specifically expressed in differentiating HCs at early embryonic stages and is absent from SCs and from cells outside the sensory epithelium in the cochlea.
These data support *Math1* as an essential factor in the control of HC differentiation. To determine whether expression of *Math1* was required for the roscovitine-induced appearance of supernumerary HC, the inventors used of cultured organs of Corti from *Math*^{*-*/-} mice. In *Math1-*null mice, the treatment of E15.5 organs of Corti with roscovitine does not induce the appearance of HCs after 5DIV. However, in heterozygote mice, □-gal positive supernumerary HCs arose. Taken together, these results demonstrate that the induction of HCs by roscovitine likely recapitulates the developmental pattern of HC development which is drastically *Math1*-dependent.
The invention has then for object to provide means for treating pathologies associated with dysfunction of the development of the organ of Corti, particularly deafness.
It particularly relates to the use of at least one kinase inhibitor for making drugs for inducing differentiation of supernumerary hair cells and Deiters'cells in the developing organ of Corti.
Said kinase inhibitor(s) is (are) particularly a kinase inhibitor selected in the group comprising purine derivatives, such as disclosed in US patent 6 316 456, preferably the so-called roscovitine, indirubins and purvulanols, and the pharmaceutically acceptable acid addition salts thereof.
Pharmaceutically acceptable acid addition salts of said compounds are formed with organic or inorganic acids according to usual methods.
Suitable acids comprise acetic, ascorbic, maleic, phosphoric, salicylic and tartric acids.
The drugs are administered to a patient in need thereof in an efficient amount and in association with a pharmaceutically acceptable carrier.
Said carrier may be solid or liquid depending on the administration form.
The medicaments can be administered in various forms : parenterally, rectally, topically, transdermally or orally. They are more particularly administered by the oral or injectable route.
For administration by the oral route, lozenges, compressed tablets, pills, tablets, capsules, drops, syrups, suspensions or emulsion, may be used. These compositions advantageoulsy comprise 100 to 1000 mg of active principle per dose unit, preferably 300 to 600 mg.
Other forms of administration include injectable solutions for the intravenous, subcutaneous or intramuscular route, formulated from sterile or sterilizable solutions. They can also be suspensions or emulsions.
These injectable forms comprise 100 to 1000 mg of said compound, or a pharmaceutically acceptable salt thereof, preferably 300 to 600 mg, per dose unit.

The invention also relates to a method for inducing differentiation of supernumerary hair cells and Deiters'cells in the developing organ of Corti comprising administering to a patient in need thereof an efficient amount of at least one kinase inhibitor as above defined, or a pharmaceutically acceptable acid addition salts thereof, in association with a pharmaceutically acceptable carrier. Other characteristics and advantages of the invention will be given hereinafter with reference to the figures which represent, respectively,
- Figure 1: Schematic representation of the structure of the cultured organ of Corti: Cross-sectional representation (B) and corresponding surface view (A) of the sensory epithelium at E19 as observed in organotypic explant cultures. **IHC**, inner hair cell; **O1-O3**, outer hair cells; **d1-d3**, Deiters' cells; **t1-t2**, tectal cells; **h1-h4**, Hensen's cells; **ut**, undertectal cells; **p**, pillar cells; **ipc**, inner phalangeal cells; **bc**, border cells.Color codes of A refer to B cell subtypes.

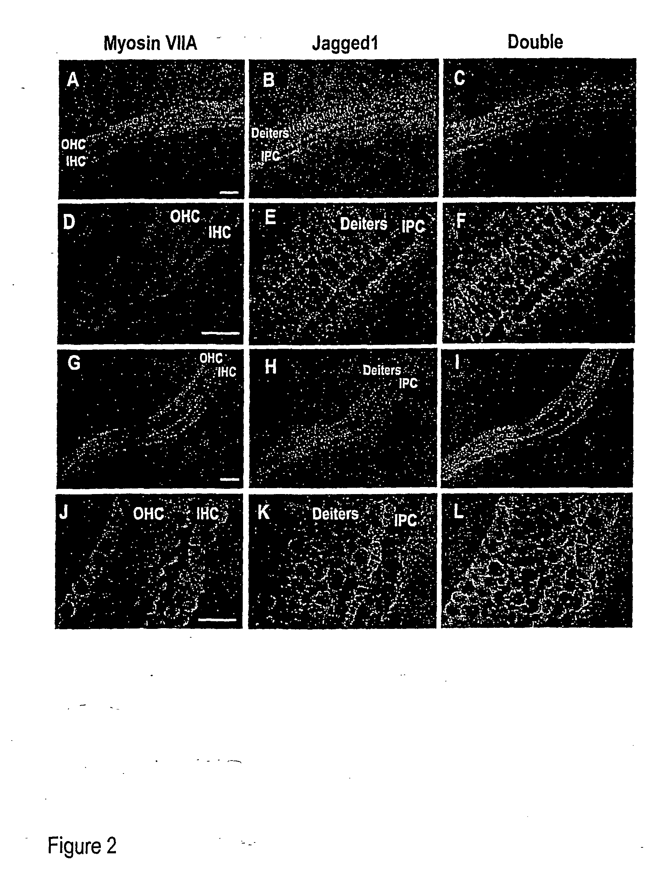

- Figure 2: Hair cells and Deiters' cells development in E19 rat organs of Corti following 5 days of culture in control medium (A-F) or in the presence of 10 :M roscovitine (G-L): The expression patterns of Myosin VIIA (A,D,G,J) and Jagged1 (B,E,H,K) in control (A-F) and roscovitine treated (G-L) E19 rat organs of Corti at 5DIV was investigated by immunolabeling and confocal microscopy. A-C, D-F, C-I, J-L represent single microscopic fields visualized in z-sections stack by confocal imaging. Scale bars = 15: m for all panels.

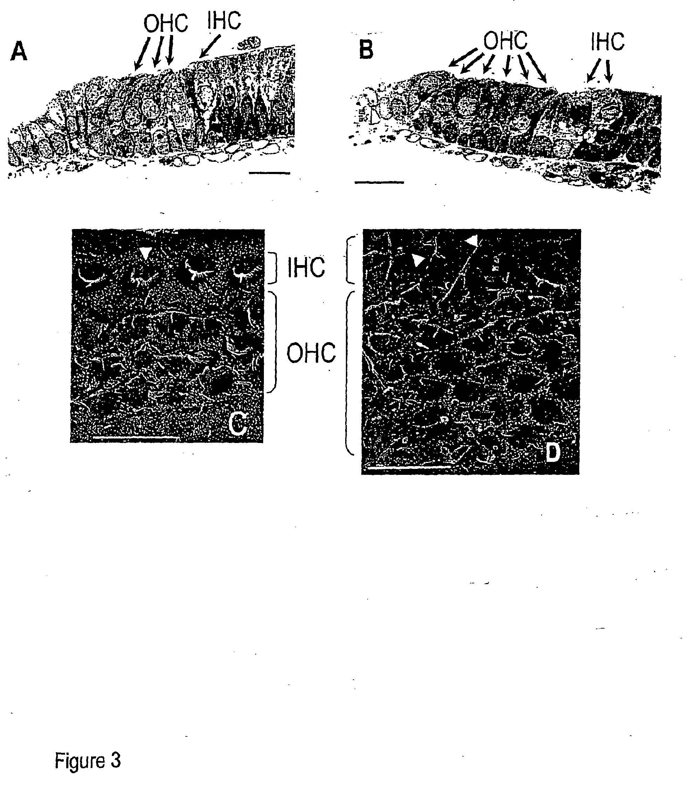

- Figure 3: Roscovitine triggers the appearance of supernumerary Hair cells: Organs of Corti from E19 rat embryos were cultured for 5 days in the absence (A, C) or presence (B, D) of 10 :M roscovitine. Explants were analysed by photonic microscopy after toluidine blue -staining of semi-thin sections (A, B) or by scanning electron microscopy (C, D). In controls (C) hair cells are arranged in three orderly rows for outer hair cells (OHC) and a single row for inner hair cells (IHC), showing uniform orientation of their stereociliary bundles. In roscovitine-treated explants (D) many supernumerary OHCs were present while the IHCs were barely visible due to regrowth of a tectorial membrane. Bar = 15 :m for all panels.

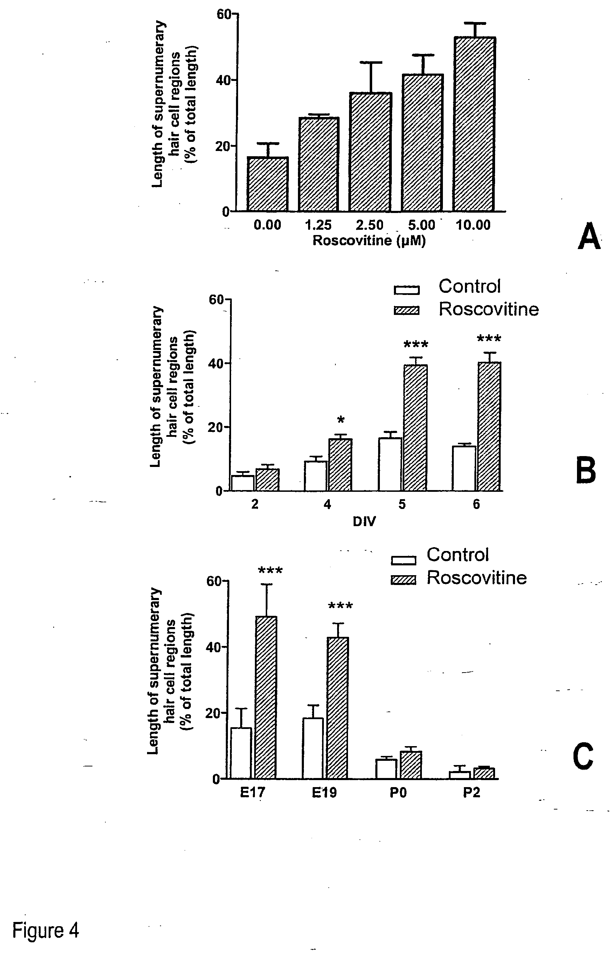

- Figure 4: Roscovitine effects: dose-response (A), kinetics (B) and developmental stage specificity (C): (A) dose-response curve for roscovitine-induced supernumerary HCs in E19 cultured organ of Corti explants. Each data bar represents the mean length of supernumerary HCs regions for a minimum of 4 explants per experiment. (B) The mean length of supernumerary HCs regions in control condition or in the presence of roscovitine (10 :M) was calculated as a function of time in culture with E19 rat organ of Corti explants. (C) Developmental stage dependence of roscovitine-induced supernumerary cells. Ten :M roscovitine was added to rat organ of Corti explants dissected from stages E17, E19, P0, P2 and P4. The mean length of supernumerary HCs regions was monitored after 5 days of culture. Results were expressed as mean ∀□ sem (n = 5). Statistical significance was determined using a Student's *t*-test. * = p< 0.05 and *** = p<0.001.

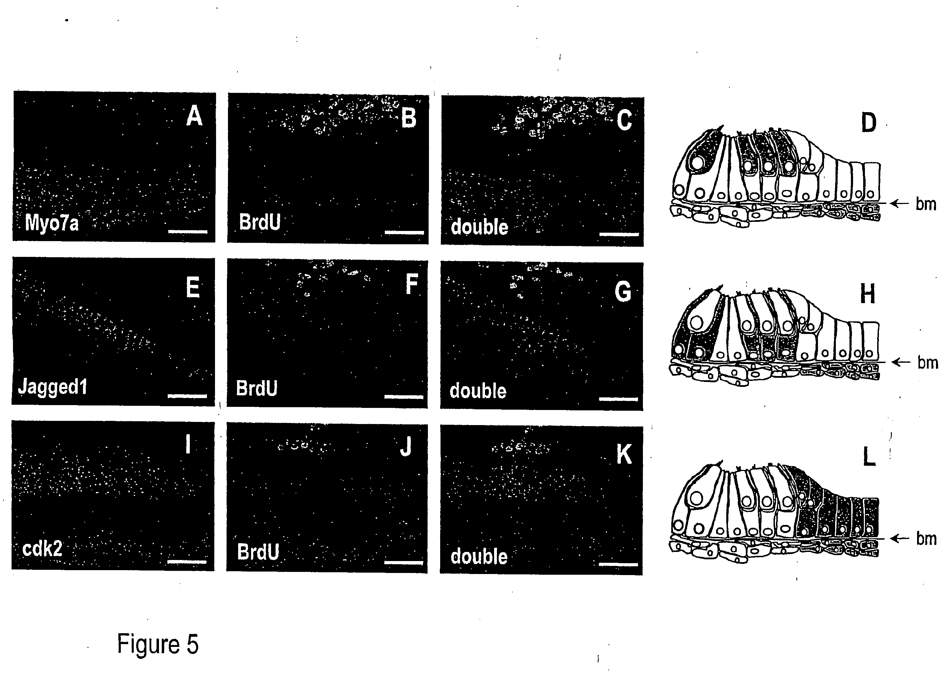

- Figure 5: Roscovitine does not interfere with cell proliferation which is absent in the organ of Corti: Confocal images of the expression pattern of Myosin VIIA (A,C), jagged1 (E,G) and cdk2 (I,K) double labeled with BrdU (B,C,F,G,J and K) in whole-mount explants of E19 cochleas after 5DIV showing that none of these markers co-localized with BrdU positive cells that were only located in connective tissues. Z-series of the entire thickness of the explant were saved and recombined to produce a single image. (D,H,L): schematic drawings depicting a cross-sectional view of the cytoarchitectural structure of organs of Corti for each ligne of immunostaining. Red-colored cells represent alternatively myosin VIIA-positive cells (D), jagged 1-positive cells (H) and cdk2-positive cells (L); BM = basal membrane; green cells represent the location of dividing cells (i.e. BrdU positive) that were all observed outside the sensory epithelium. Scale Bar= 15 :M for all panels.

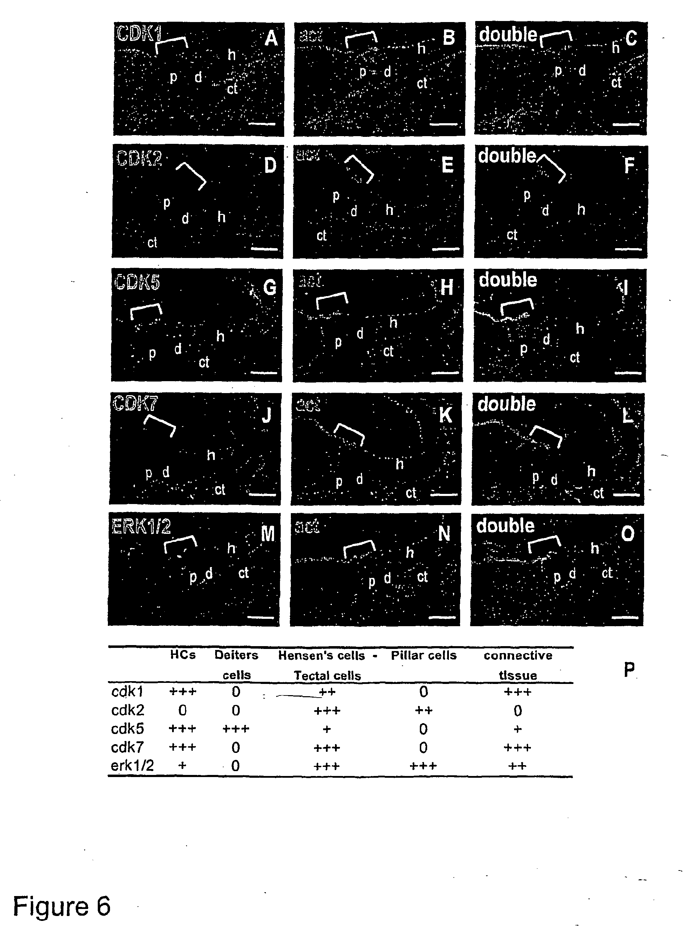

- Figure 6: In situ immunohistochemical localization of CDK1, CDK2, CDK5, CDK7 and ERK1/2 in E19 rat organs of Corti: E19 rat organ of Corti sections immunostained with anti-CDK1 (A,C), anti-CDK2 (D,F), anti-CDK5 (G,I), anti-CDK7 (J,L), anti-ERK1/2 (M,O) antibodies and phalloidin-TRITC (∃-actin in B,C,E,F,H,I,K,L,N). Recapitulative table of immunopositive cell phenotypes for each protein (P). Brackets = HCs, p = Pillar cells, d = Deiters' cells, h = Hensen's cells, ct = connective tissue, act = ∃-actin.

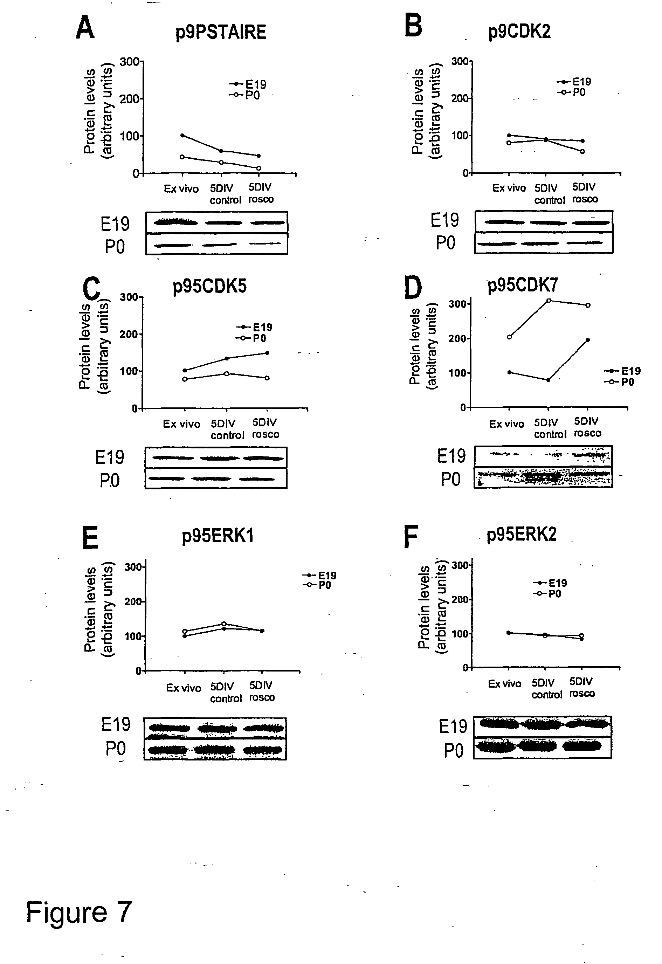

- Figure 7: Kinases expression in E19 and P0 organs of Corti explants ex vivo or cultured with or without 10 :M roscovitine. Levels of protein expression for CDK1/2 (A), CDK2 (B), CDK5 (C), CDK7 (D), ERK1 (E) and ERK2 (F) were analysed by Western immunoblot analysis at E19 and P0 after affinity purification on p9^{CKShs1} or aminopurvalanol (p95) beads. Organs of Corti were dissected from E19 and P0 rats and protein levels were quantified directly (ex vivo condition) or after 5DIV in the presence or absence of 10 :M roscovitine. Results were expressed as percentages of protein levels observed in E19 ex vivo explants.

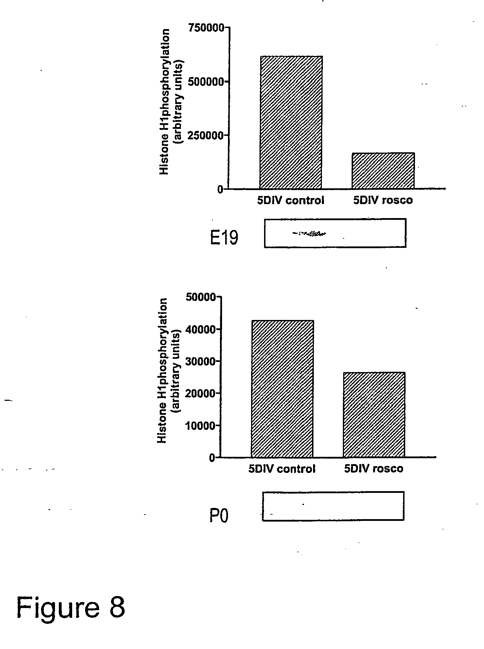

- Figure 8: Effect of treatment with 10 :M roscovitine on CDK1/2 activity in E19 and P0 cultured organs of Corti after 5DIV. Kinase assays were performed using histone H1 as a substrate in the presence of 15 :M ATP. Reactions were stopped by addition of 40 :1 of Laemmli sample buffer. Samples were boiled and separated by 10% SDS-PAGE. Gels were diced and subjected to autoradiography. Densitometric analysis was performed using Image Master 1D software.
- Figure 9: Math1 is necessary for hair cell production induced by roscovitine. Surface views at the middle turn of an E18 *Math1* ^{+/+} (A), an E15.5 *Math1* ^{∃*_*gal/∃_gal} (B) and an E15.5 *Math1* ^{+/∃gal} (C) cochleas in culture for 5DIV in the presence of roscovitine (10:M). Myosin VIIA immunostaining is represented in brown and 3-gal staining is in blue. Bar = 15:m.

### EXPERIMENTAL PROCEDURES

### Culture and treatments of organs of Corti

Time-pregnant Sprague-Dawley rats or neonates were killed by euthanasia on gestational days 17 (E17), E19, or on postnatal days 0 (day of birth = P0), P2 and P4 with CO₂. Both cochleae were dissected free from the calvaria with watchmaker forceps and under a stereomicroscope. Each organ of Corti was freed from surrounding tissues and explanted intact onto the surface of a sterile membrane (Millicell^{™}, 12 mm, Millipore, Bedford, MA, USA) in minimum essential medium (MEM; Gibco, Gent, Belgium) into a 24-well culture plate (Nunc). Cultures were incubated at 37°C in a humidified atmosphere of 95% air and 5% CO₂. Medium was renewed every 2 days. (R)-Roscovitine (Alexis Corporation, San Diego, USA) was dissolved as a stock solution in DMSO and added as indicated.

### Generation of Math1 knock-in mice

*Math1* knockout mice were generated by replacing the *Math1* open reading frame (ORF) with the selectable marker PGKhprt as previously described by Ben-Arie et al.(1). *Math1*^{∃_gal/∃_gal} knock-in mice were generated by replacing the *Math1* ORF with a ∃-galactosidase (∃-gal) reporter gene so that 3-gal is expressed in all cells that express *Math1* (2).

### LacZ staining and genotyping of embryos

Whole-mount *lacZ* staining was carried out on cultured E15.5 organ of Corti explants as previously described (2;3). After 5DIV, explants were fixed in 4% paraformaldehyde for 20 minutes, washed thoroughly in PBS and equilibrate in X-gal staining buffer (5 mM potassium ferricyanide, 5 mM potassium ferrocyanide, 0.02% NP40, and 0.01% sodium deoxycholate). Explants were stained overnight in X-gal buffer with X-gal (1 mg/ml) then post-fixed in 4% paraformaldehyde. Genotyping of tail DNA was performed using Southern analysis of EcoRI digested DNA and probes as previously described (1).

### Semithin sections

Explants were fixed in 2.5% glutaraldehyde in 0.1M sodium cacodylate buffer (pH 7.4) for 45 min. Specimens were then post-fixed in 1% OsO₄ in the same cacodylate buffer for 30 min., dehydrated in graded ethanol concentrations and propylene oxide and embedded in epoxy resin (Agar Scientific Ltd, UK). Organ of Corti explants were sectioned parallel to the longitudinal axis of the HCs. Serial semi-thin sections, 1 :m-thick, were cut with a diamond knife, stained with toluidine blue (0.5%) and observed by light microscopy.

### Scanning electron microscopy

Explants for surface ultrastructural analysis were fixed in 2.5% glutaraldehyde in 0.1 M sodium cacodylate (pH 7.2) for 2 hours, washed three times in cacodylate buffer, then postfixed for 1 hour with 1% osmium tetroxide. Explants were dehydrated in ascending concentrations-of ethanol, critical-point dried from liquid CO₂ and sputter coated with gold. All material was examined in a JEOL JSM-840 scanning electron microscope operating at 20 kV.

### Immunohistochemistry, immunocytochemsitry, and confocal microscopy

Immunohistochemistry, immunocytochemistry and confocal microscopy were carried out as previously described (4;5). Whole-mount preparations or cryostat sections (10-20 :m) were fixed with either 4% paraformaldehyde (10 min. at 20°C) or methanol (5 min. at -20°C). Primary antibodies incubations were performed overnight at 4°C. Preparations were washed three times in PBS before incubating with a secondary antibody conjugated to alexa488, alexa568 or TRITC fluorophore for 1 hour at 20°C. Sequential double labeling experiments were done with a 4% paraformaldehyde fixation step in between each immunoreaction. Preparations were mounted on microscope slides and coverslipped with Fluoprep mounting medium (BioMérieux, Marcy l'Etoile, France) and imaged using a Bio-Rad MRC1024 laser scanning confocal microscope. To label cells in S phase in vitro, BrdU (10 :M, Sigma, USA) which is incorporated into replicating DNA, was added to the cultures for 2 or 5DIV prior staining.

The following primary antibodies were used: anti-myosin VIIa (1:200; Tama Hasson, University of California at San Diego, San Diego, CA, USA), anti-CDK1 (1:100, SC-54, Santa Cruz Inc., USA) anti-CDK2 (1:100, SC-163, Santa Cruz), anti-CDK5 (1:100, SC-173, Santa Cruz), anti-CDK7 (1:100, SC-529, Santa Cruz), anti-ERK1 (reactive with ERK1 and ERK2, 1:100, SC-94, Santacruz), anti-Jagged1 (1:100, SC-6011, Santa Cruz), anti-BrdU-FITC-conjugated antibody (1:3, Becton-Dickinson, USA). Omission of primary antibodies resulted in a complete loss of immunostaining.

### In situ detection of apoptosis: TUNEL labelling

The apoptosis in E19 rat organ of Corti explants was looked for by staining the specimens cultured for 1, 2 or 5DIV with the terminal deoxynucleotidyl transferase-mediated dUTP nick-end labelling (TUNEL) method using the ApopTag fluorescent detection kit (Oncor, Gaitherburg, MD) as previously described (6).

### Dose-response experiments and temporal dependence

Organs of Corti were dissected from E19 rats and maintained in culture. At the time of seeding, the culture medium contained roscovitine at 1.25, 2.5, 5, 10, 30 or 100 :M. After 5DIV, cultures were fixed and immunostained for myosin VIIa and jagged1 as described above. In order to establish the time for optimal appearance of supernumerary HCs, E19 rat organs of Corti were treated with 10 :M roscovitine and the fraction of supernumerary zone was determined following fixation and immunocytochemistry for myosinVIIa of the explants at 2, 4, 5 and 6 DIV. E17, E19, P0, P2 and P4 rat organs of Corti were dissected and cultured for 5DIV in the presence of 10 :M roscovitine. For each condition, the explants were fixed and the supernumerary zones were counted after myosin VIIa immunostaining.

### Quantification of HCs within the organ of Corti

Quantitative analysis of supernumerary HCs production was obtained by counting the number of HCs per length and surface of sensory epithelium regions from the medium turn as previously described (5). If supernumerary cells were actually produced, the number of cells per unit of length should increase while the number of cells per surface unit should remain at the same level. After showing that new cells effectively arose in our cultures conditions, the extent of supernumerary HCs was determined by measuring the length of the supernumerary zone on images obtained from the confocal microscope. The total length of each organ of Corti explant was measured and the extent of the region with supernumerary HCs was expressed as percent of total length. These supernumerary regions were defined by the presence of more than four rows of HCs.

### Western blot analysis

Cultured organs of Corti from 10 cochleae for each experimental condition were lysed in homogenisation buffer containing of 60 mM ∃-glycerophosphate, 15 mM p-nitrophenyl phosphate, 25 mM MOPS (pH 7.2), 15 mM EGTA, 15 mM MgCl₂, 1 mM DTT, 1 mM sodium orthovanadate, 1 mM sodium fluoride, 1 mM phenyl phosphate, 10 :g/ml leupeptin, 10 :g/ml aprotinin, 10 :g/ml soybean trypsin inhibitor and 100 :M benzamidine. After sonication, insoluble material was removed by centrifugation at 12 000 g for 15 minutes at 4°C. Protein concentration was determined by the Bradford assay (Biorad). For crude extract analysis, proteins were heat denaturated in Laemmli sample buffer and resolved by 10 % SDS-PAGE (0.75 mm thick gels) followed by transfer on 0.1 :m nitrocellulose filters. These were blocked with 5 % low fat milk in Tris-Buffered Saline-Tween-20 for 1 hour at room temperature, then incubated 1 hour with anti-PSTAIRE (1:3000, P7962, Sigma);-anti-CDK5 (1:500, SC-173, Santa Cruz), anti-CDK7 (1:500, SC-529, SantaCurz) or anti-ERK1 (1:4000, M7927, Sigma), and analyzed by Enhanced Chemiluminescence (ECL, Amersham). Quantification of the results was carried out by densitometric analysis using Image Master 1D (Amersham-pharmacia, Belgium).

### Affinity matrices

Purvalanol (NG-95) and p9^{CKShS1} affinity matrices were synthesized as described (7;8;9;10). Just before use, 10 :1 of settled beads were washed in 1 ml of Bead Buffer (50 mM Tris pH 7.4, 5 mM NaF, 250 mM NaCl, 5 mM EDTA, 5 mM EGTA, 0.1% Nonidet P-40, 10 :g/ml leupeptin, 10 :g/ml aprotinin, 10 :g/ml soybean trypsin inhibitor, 100 :M benzamidine) and resuspended in 400 :1 of this buffer. Cell extracts prepared as described above were then added (1 mg total protein) and the tubes rotated at 4°C for 30 minutes. After a brief spin, the supernatant was removed and the beads were washed four times with 1 ml Bead Buffer before addition of 50 :1 of Laemmli sample buffer. Following heat denaturation for 3 minutes, the bound proteins were resolved by SDS-PAGE and analyzed by Western blotting as described above.

### Quantification of CDK activity

p9^{CKShs1}-sepharose affinity purification was also used to determine the histone H1 kinase activity of bound CDK1 and CDK2. After purification as described above, the p9^{CKShs1}-sepharose kinases were incubated for 30 min at 30°C with 1 □Ci [³²P] ATP (1-3 Ci/mmol, Amersham) in the presence of 25 µg histone H1 (Type III-S, Sigma) in a final volume of 30 µl of buffer C (homogenization buffer but 5 mM EGTA, no NaF and no protease inhibitors). Assays were terminated by transferring the tube on ice. 30 :1 2x Laemmli sample buffer was added. Phosphorylation of the substate was assessed by autoradiography after SDS-PAGE.

### RESULTS

### Induction of supernumerary hair cells by roscovitine and by other kinase inhibitors

Cochlear explants from E19 rat embryos developed a normal sensory epithelium after 5 days in culture with a single row of inner hair cells and three rows of outer hair cells, both being specifically stained with myosin VIIa (Figure 2 A, D). Inner and outer HCs are supported, respectively, by one row of inner phalangeal cells (IPC) and three rows of Deiters'cells, both specifically stained with anti-jagged1 antibodies as previously described (11) (Figure 1, Figure 2 B, E). Cultures treated during 5 days with 10□M roscovitine developed a higher number of supernumerary HCs and corresponding SCs (i.e. Deiters' cells and IPC) organized in rows as in control conditions (Figure 2G-L). The supernumerary regions were characterized by four to nine rows of outer HCs, two to three rows of inner HCs and six to twelve rows of the corresponding SCs. However, all the roscovitine-induced supernumerary HCs were still separated from each other by intervening SCs. The production of supernumerary HCs was confirmed with photonic microscopy of sections through the organ of Corti and with scanning electronic microscopic studies (Figure 3).

To ascertain whether the effect of roscovitine was mediated by the inhibition of specific kinases, a series of chemically related and unrelated CDK inhibitors (Table 1) was tested.
**Table 1. Induction of supernumerary HCs by various kinases inhibitors.** Compounds were tested at various concentrations for their ability to induce supernumerary HCs in isolated organs of Corti. The lowest concentration inducing maximum effect is presented. For inactive compounds the highest concentration tested is shown in parentheses. In addition to CDK inhibitors, the MAP kinase kinase inhibitor U0126 and the transcription inhibitors actinomycin D and DRB were also tested. The IC₅₀ values of all compounds for CDK1/cyclin B inhibition is also provided. * = maximum concentration tested.

| | **Target** | **Concentration inducing maximum supernumerary HCs differentiation (:M)** | **CDK1 inhibition (IC₅₀, :M)** (Knockaert et al, 2002b) |
|---|---|---|---|
| **olomoucine** | CDK1, 2,5 and 7 and MAPK pathway | 30 | 7 |
| **roscovitine** | CDK1, 2, 5 and 7 and MAPK pathway | 10 | 0,45 |
| **aminopurvalanol** | CDK1, 2, 5 and 7 and MAPK pathway | 5 | 0,004 |
| **iso-olomoucine** | non-effective analogue of olomoucine | no effect (100:M)* | > 500 |
| **6-methyl-aminopurvalanol** N6-methylaminopurvalanol | non-effective analogue of aminopurvalanol | no effect (100 :M)* | > 100 |
| **alsterpaullone** | CDK1, 2, 5 and 7 and MAPK pathway | 5 | 0,035 |
| **indirubin-3'-monoxime** | CDK1, 2, 5 and 7 | 10 | 0,18 |
| **fascaplysin** | CDK4 and 6 | no effect (100 :M)* | no effect |
| **U0126** | MAPK pathway | no effect (100 :M)* | no effect |
| **Actinomycin D** | Transcription inhibition | 0,0001 (toxic at higher concentrations) | ?? |
| **DRB** | Transcription inhibition | 0,01 (toxic at higher concentrations) | ?? |

The 2,6,9 -tri-substituted purines were all able to induce the appearance of supernumerary HCs. Their efficiency in inducing this effect on isolated organs of Corti correlated with their potency in inhibiting CDKs (aminopurvalanol > roscovitine > olomoucine). The kinase inhibitors' inactive analogues, methyl-aminopurvalanol (8;10) and iso-olomoucine (12) had no effect on the isolated organs of Corti (Table 1). Other, chemically different, CDK1/2/5 inhibitors such as indirubin-3'-monoxime (13) and alsterpaullone (14) also induced the appearance of supernumerary HCs while the CDK4/6 inhibitor, fascaplysin (15) had no effect.

Since roscovitine, and other CDK inhibitory purines inhibit CDK7, that is involved in the activation of transcription, two inhibitors of transcription: 6-dichloro-1-beta-D-ribobenzimidazole (DRB) and actinomycin D were tested(16;17). Both actinomycin D and DRB were able to induce supernumerary HCs (Table 1).

Roscovitine, and other CDK inhibitory purines have been shown to inhibit the MAP kinases ERK1 and ERK2 *in vitro* and *in vivo.* The MAPK pathway inhibitor, U0126 (18) was then used. Even at concentrations as high as 100 :M , U0126 did not induce supernumerary HCs (Table 1).

### Dose-dependence, time-course and developmental regulation of roscovitine-induced supernumerary HCs

In order to rule out a possible rearrangement of the HCs in the organ of Corti due to a compression of the explant or to a migration of the sensory cells, we assessed the number of HCs per surface unit and length unit in control and roscovitine-treated explants were assessed). The results showed that the density of HCs per surface unit in roscovitine-treated explants remained identical to that of control explants. These data clearly demonstrated that supernumerary HCs arose from an actual increase in number rather than from a rearrangement of the existing HCs. The length of E19 rat organs of Corti explants containing supernumerary HCs increased as the concentration of roscovitine was raised (Figure 4A). At 10 :M roscovitine and after 5DIV, supernumerary segments represented 52.9 % V 4.3 (mean V S.D., n = 4 independent experiments) of the total length of the explants. At higher concentrations (30 and 100 :M) roscovitine was found to be toxic for all cells in the organ of Corti. In contrast the average thickness (number of HCs rows) of supernumerary HCs segements was also significantly higher in roscovitine-treated vs. control explants.

The effect of roscovitine on the development of supernumerary HCs was directly related to the duration of treatment (Figure 4B). The average length of supernumerary zones in the E19 rat organ of Corti explants maintained in the presence of 10 :M roscovitine increased progressively with time in culture, yielding a significant effect after 4DIV treatment, and a maximal effect subsequently to a 5DIV. The spontaneous occurrence of supernumerary HCs in cultured organotypic explants of organ of Corti decreased from E17 to P0 and this process totally vanished at P2 (figure 4C). Likewise, the effect of roscovitine on the induction of supernumerary HCs culminated at E17, sightly decreased at E19 and disappeared at P0.

### Mechanism of roscovitine-induced supernumerary HCs does not rely on the regulation of cell proliferation and apoptotic cell death

Roscovitine, like other CDK inhibitors, has potent anti-mitotic properties (reviewed in (19;20). Cell proliferation in E19 explants of organ of Corti treated with roscovitine was then investigated. Explants were incubated for either 2 or 5 DIV in the presence of 10 :M BrdU. No epithelial cells were labeled with BrdU in the area of HCs and SCs (Figure 5). Confocal analyses displayed mitotic figures that were observed exclusively in connective tissues of mesenchymal origin, while no BrdU positive cells were found within the planes of the sensory epithelium (figure 5). The rate of proliferation of mesenchymal cells located in connective tissue areas was actually higher in the absence of roscovitine consistent with its well established effect on cell cycle progression.

To determine whether apoptosis was implicated in HCs and SCs differentiation, E19 rat organ of Corti explants were double-stained for DNA fragmentation (TUNEL positive) and for f-actin in the stereocilia bundles and cuticular plate. After 1, 2 or 5 DIV, no TUNEL positive nuclei were identified either in control or roscovitine (10 :M) treated cultures (data not shown). The evidence that supernumerary HCs and corresponding SCs occur in the absence of cell division and apoptosis thus strongly supports the hypothesis of a direct differentiation of pre-existing immature progenitors cells into both HCs and corresponding SCs.

### Immunohistochemical localization of molecular targets of roscovitine in the E19 rat organ of Corti

In order to characterize the molecular components of the transduction cascade underlying the effect of roscovitine on supernumerary cells, we first studied the expression of CDK1, CDK2, CDK5, CDK7 and ERK1/2 by immunohistochemistry using sections obtained from E19 rat organ of Corti (Figure 6). CDK1, CDK5, CDK7 and ERK1/2 were shown to be ubiquitously expressed throughout the organ of Corti, including the HCs (figure 6P). Interestingly, CDK2 was found to be specifically present in tectal cells, Hensen's cells (h in figure 6D-F) and to a lesser extent in pillar cells in E19 explants of organs of Corti. The specific expression pattern of CDK2 persisted throughout the early postnatal period (data not shown). It is important to emphasize that cells immuno-positive for myosin VIIA, i.e. HCs, or for jagged1, *i.e.* Deiters' cells and IPC, were never immunoreactive for CDK2 (data not shwon).

### Expression of molecular targets of roscovitine in the organ of Corti ex vivo and in vitro

The levels of expression of CDKs and ERKs following affinity purification (Figure 7) was then investigated and the levels of protein expression in organs of Corti were compared between two developmental stages, i.e. E19 and P0, which are respectively sensitive and insensitive to roscovitine for the induction of supernumerary HCs. E19 and P0 rat organs of Corti were dissected, lysed and CDK1, CDK2 and their associated proteins were purified by affinity chromatography on p9^{CKShs1} beads, the mammalian homologue of CKS1 from *Saccharomyces cerevisiae* and p13^{suc1} from *Schizosaccharomyces pombe.* The expression of CDK1/2 was assessed by immunoblotting analysis using PSTAIRE specific antibody while the expression of CDK2 alone was evaluated by a specific anti-CDK2 antibody. As shown in Figure 7A, the expression of CDK1/2 diminished between E19 and P0 for each condition (ex vivo explants and cultures with or without roscovitine). In contrast the level of CDK2 remained more constant (figure 7B) suggesting that the expression of CDK1 was lower at P0 ex vivo and that CDK1 was drastically down-regulated in vitro especially in the presence of roscovitine both with E19 and P0 organs of Corti.

Affinity chromatography on immobilized purvalanol which sensitizes the recovery of CDK5, CDK7, ERK1 and ERK2, that are also the specific targets of the 2,6,9-trisubstituted purines was used. The expression of CDK5 ex vivo was slightly higher at E19 ex vivo and increased in culture only in explants isolated from E19 organs of Corti both in control condition and in the presence of roscovitine. diminished between E19 and P0 for each condition, i.e. explants and cultures in the presence or not of roscovitine (figure 7C). The expression of ERK1 and ERK2 was not different between E19 and P0 ex vivo and remained stable in culture (figure 7E and 7F). In contrast, CDK7 expression was much higher in P0 organs of Corti ex vivo (figure 7D).

### Changes in CDK1/2 activities in E19 and P0 rat organs of Corti following roscovitine treatment

The changes in CDK1/2 activities occuring in lysates of E19 and P0 organs of Corti during roscovitine-induced HCs and SCs differentiation (roscovitine 10 :M) (Figure 8) was examined. p9^{CKShs1}-sepharose affinity purified lysates were assessed for their ability to phosphorylate histone H1. As shown in Figure 8, kinase activities in control conditions was higher in cultures from E19 organs of Corti, consistent with data from Western blot analysis. Furthermore, in the presence of roscovitine (10 :M) a significant inhibition of CDK1/2 kinase activity that was relatively more important with E19 cultured organs of Corti was observed.

### Induction of supernumerary HCs and SCs by roscovitine requires the transcription factor Math1

Further experiments were carried out to investigate whether the transcription factor Math1 was upstream or downstream of the roscovitine effect, using of the Math1 -/- mice (Bermingham et al, 1999). When organs of Corti dissected from E15 or E18 wild type mice were exposed to roscovitine for 5DIV myosin VIIA immunopositive supernumerary HCs, (Figure 9A) were observed. Myosin VIIA staining was absent in the cochlea of E15.5 *Math1-*null mutant mice *(Math1* ^{*∃*gal/*∃*gal}) cultured for 5DIV in control medium or in the presence of roscovitine (Figure 9B). In *Math1* ^{+/*∃*gal} mice, it has been previously shown that ∃-gal was restricted to the HCs of the developing sensory epithelia (Bermingham et al., 1999). After culturing E15.5 *Math1* ^{+/}*^{∃gal}* organs of Corti for 5DIV in the presence of roscovitine at 10 :M, supernumerary HCs were present and identified by a myosin VIIA positive staining (figure 9C). These supernumerary myosin VIIA positive cells are also ∃-gal positive, i.e. *Math1* positive.

### DISCUSSION

The mammalian organ of Corti is a one of a kind sensory epithelium characterized by a highly organized cytoarchitecture, with HCs and SCs arranged in a precise alternative pattern. Studying of how their production is controlled during development is crucial towards understanding the causes of deafness or attempting to induce HC regeneration. In rodents, auditory HCs and SCs precursors have their terminal mitosis between E13 and E17. However, a production of supernumerary HCs has been shown to occur after the ontogenesis of the sensory cells, in various circumstances. Previous studies reported the production of supernumerary HCs in embryonic cochlear explant cultures following retinoic acid treatment, in normal serum-free medium or in mice in which a specific gene is deleted, such as p27^{KIP1} jagged-2, or HES transcription factors.
The results provide clear evidence that roscovitine and other pharmacological inhibitors of protein kinases induce the production of a large number of supernumerary HCs and corresponding SCs (i.e. Deiters' cells and IPC) in E19 rat organs of Corti.

### Roscovitine-induced supernumerary HCs have all the features of preexisting HCs

Evidence based on myosin VIIA staining and scanning electron microscopic observations showed that all HCs, including supernumerary HCs generated in the presence of roscovitine, shared the same phenotype in E19 rat organs of Corti explants cultured for 5 days. In addition, in supernumerary zones, the normal cytoarchitecture of the organ of Corti was conserved, as no area displaying only HCs without interposed SCs was never observed. Taken together, these results showed that progenitor cells which have retained the capacity to become HCs or corresponding SC can be induced by roscovitine to differentiate into newly formed cells according to a developmental program that likely recapitulates the events occuring during normal ontogenesis.

### CDK inhibition triggers a phenotypic differentiation of precursor cells into supernumerary HCs and corresponding HCs without interacting with cell cycle progression and apoptosis

The activity of CDKs critically contributes to the molecular switch between cell proliferation and differentiation in several cell types Roscovitine has been described to induce cell differentiation through its inhibition of cell cycle progression leading to an arrest of proliferation. Such a mechanism is not involved in the production of supernumerary HCs and corresponding SCs because no cell proliferation was ever observed in cultured intact sensory epithelium from E19 rats. Roscovitine has also been described as a pro-apoptotic agent in undifferentiated cells mostly by blocking the cell cycle or an anti-apoptotic agent on differentiated cells such as neurons.
TUNEL studies revealed no apoptotic cells within the sensory epithelium after 5 days of culture, both in control or roscovitine-treated conditions, excluding that a possible interaction of roscovitine with apoptotic processes may partly underlie the production of supernumerary HCs and corresponding SCs. The absence of proliferation and apoptosis in the presence of roscovitine within the organ of Corti argues in favor of a direct phenotypic conversion of pre-existing cells into HCs and corresponding SCs. Cell conversion has been observed in amphibian lateral line organ after laser ablation of HCs, in amphibian vestibular sensory epithelium after aminoglycoside-induced HC damage in avian basilar papilla after noise trauma or aminoglycoside damage and in utricular macula of mature guinea-pigs following gentamicin-induced HC death.

### The targets of roscovitine in the organ of Corti

Roscovitine is a pharmacological inhibitor of protein kinases which has been tested on over 25 kinases and possesses a high specificity for CDK1, 2, 5, 7 and the MAP kinases ERK1 and ERK2 . Experiments were carried out to study whether the generation of supernumerary HCs and corresponding SCs in the organ of Corti was the result of the inhibition of any specific roscovitine's kinase targets. Roscovitine and other 2,6,9-tri-substituted purines, i.e. olomoucine and aminopurvalanol, are known to inhibit ERK1 and ERK2. However, U0126 which is a specific antagonist of the MAP Kinase pathway, did not induce the appearance of supernumerary cells. These data suggest that inhibition of ERK1/2 is unlikely to participate to the mechanism of action of roscovitine on the isolated organ of Corti. Fascaplysin, a CDK4 and CDK6 specific inhibitor, did not trigger the appearance of supernumerary cells. Furthermore, all other CDK inhibitors used in this study have no effect on CDK4, and yet triggered the appearance of supernumerary HCs and corresponding SCs (Table 1). Altogether this supports the view that CDK4/6 inhibition is not required for supernumerary HCs and corresponding SCs differentiation. Roscovitine analogues non-structurally related such as alsterpaullone and indirubin-3'-monoxime which are CDK1,2,5,7 inhibitors like roscovitine but devoid of effect on the MAP Kinase pathway also induced supernumerary HCs and corresponding SCs. Altogether, this demonstrates that roscovitine stimulates the differentiation of supernumerary cells by the inhibition of a subset of CDKs, i.e. CDK1, CDK2, CDK5 and CDK7. *Hensen's cells, the roscovitine-sensitive HCs and corresponding SCs progenitor cells ?*

There is compelling evidence from both mammals and non-mammals that HCs and SCs share a common progenitor.
These models suggest that uncommitted cells within developing sensory epithelia essentially compete to become committed to the HC phenotype. Once an individual cell has become committed to differentiate as a HC, that cell then exerts a form of lateral inhibition on any neighboring cells that it contacts, so that the immediate neighbors of HCs are prevented from differentiating as HCs. Those inhibited cells are constrained to differentiate as Deiters cells. Indeed, in all in vitro and in vivo models, the appearance of supernumerary HCs is systematically accompanied by the production of supernumerary Deiters' cells or pillar cells while no overproduction of Hensen's cells have been described. The immunocytochemical data revealed that potential molecular targets of roscovitine, i.e. CDK1,2,5,7 are all ubiquitously expressed in the organ of Corti except CDK2 which is only expressed in Hensen's cells and to a lesser extend in pillar cells. Previous works demonstrated the role of Hensen's cells as possible progenitors for HCs and corresponding SCs. All together these results suggest that Hensen's cells are suited candidates to represent the cellular source of roscovitine-induced supernumerary HCs and corresponding SCs.

### Molecular insights from developmental regulation of roscovitine-induced supernumerary HCs and corresponding SCs

In order to untangle respective roles of CDK1,2,5,7 in the transduction pathway underlying the induction of supernumerary cells in the organ of Corti by roscovitine, levels of protein expression between two developmental stages that were permissive (E19) or repressive (P0) with respect to this phenomenon was compared. Moreover, not only spontaneous supernumerary cells were more seldom at P0 than at E19 but the stimulation of this process by roscovitine was absent at P0 and sustained a high level at E19. At different extent, CDK1,2 and 5 expression were decreased at P0 ex vivo, consistent with the lower rate of emergence of supernumerary cells and with the absence of effect of roscovitine at this stage. The fact that transcription inhibitors such as actinomycin D and DRB also induce the apparition of supernumerary HCs and corresponding supporting cells, strongly suggest that inhibition of CDK7 could be involved.

Altogether these results indicates that CDK1,2,5 and 7 are good candidates to mediate roscovitine effect on HCs and corresponding SCs differentiation.

Said results also demonstrate that CDK1/2 kinase activity was higher at E19 than at P0 and was actively inhibited by roscovitine at both stages but more severely at E19. The confirmation that roscovitine inhibited CDK enzymatic activity validated that this pharmacological agent was indeed exerting its normal biochemical properties in our in vitro model of organotypic cultures of organ of Corti.

### Math1 acts downstream of roscovitine

*Math1,* a bHLH proneural gene appear necessary for the production of HCs induced by roscovitine since in organ of Corti explants from *Math1*^{*∃*_gal/*∃*_gal} no HCs are produced in the presence of Roscovitine. In addition in heterozygote mice, *Math1* is upregulated in supernumerary HCs generated by roscovitine. These results confirm previous reports showing that *Math1* is necessary and sufficient for the differentiation of HCs in the mammals cochlea (Bermingham et al.,1999;Zheng and Gao, 2000). They demonstrate that roscovitine requires the downstream intervention of Math1 for its differentiating effect.

In conclusion, the invention demonstrates that CDKs play a role in cellular differentiation in the organ the Corti.

Roscovitine was regularly administered to patients suffering of deafness. An improvement of their troubles was observed without any drawbacks.

### Reference List

**1.** Ben Arie,N., Bellen,H.J., Armstrong,D,L., McCall,A.E., Gordadze,P.R., Guo,Q., Matzuk,M.M. and Zoghbi,H.Y. (1997). Math1 is essential for genesis of cerebellar granule neurons. Nature, 390,169-172.
**2.** Ben Arie,N., - Hassan,B.A., Bermingham,N.A., Malicki,D.M., Armstrong,D., Matzuk,M., Bellen,H.J. and Zoghbi,H.Y. (2000). Functional conservation of atonal and Math1 in the CNS and PNS. Development, 127, 1039-1048.
**3.** Bermingham,N.A., Hassan,B.A., Price,S.D., Vollrath,M.A., Ben Arie,N., Eatock,R.A., Bellen,H.J., Lysakowski,A. and Zoghbi,H.Y. (1999). Math1: An essential gene for the generation of inner ear hair cells. Science, 284, 1837--1841.
**4.** Malgrange,B., Belachew,S., Thiry,M., Nguyen,L., Rogister,B., Alvarez,M.-L., Rigo,J.-M., Van De Water,T.R., Moonen,G. and Lefebvre,P.P. (2002a). Proliferative generation of mammalian auditory hair cells in culture. Mechanisms of Development, 112, 79-88*.*
**5.** Malgrange, B., Thiry, M., Van De Water, T.R., Nguyen, L., Moonen, G., and Lefebvre, P.P. (2002c) Epithelial supporting cells can differentiate into outer hair cells and Deiters' cells in the cultured organ of Corti. CMLS (in press).
**6.**Malgrange,B., Rigo,J., Coucke,P., Thiry,M., Hans,G., Nguyen,L., Van De,W.T., Moonen,G. and Lefebvre,P. (2002b). Identification of factors that maintain mammalian outer hair cells in adult organ of Corti explants. Hear.Res., 170, 48*.*
**7.** Borgne,A. and Meijer,L. (1996). Sequential dephosphorylation of p34(cdc2) on Thr-14 and Tyr-15 at the prophase/metaphase transition. J.Biol.Chem., 271, 27847-27854.
**8.** Chang,Y.T., Gray,N.S., Rosania, G.R., Sutherlin, D.P., Kwon, S., Norman,T.C., Sarohia, R., Leost, M., Meijer, L. and Schultz, P.G. (1999). Synthesis and application of functionally diverse 2,6,9-trisubstituted purine libraries as CDK inhibitors. Chem.Biol., 6, 361-375.
**9.** Rosania, G.R., Merlie,J., Jr., Gray, N., Chang, Y.T., Schultz, P.G. and Heald,R. (1999). A cyclin-dependent kinase inhibitor inducing cancer cell differentiation: biochemical identification using Xenopus egg extracts. Proc.Natl.Acad.Sci.U.S.A, 96, 4797-4802.
**10.** Knockaert,M., Lenormand,P., Gray,N., Schultz,P., Pouyssegur,J. and Meijer,L. (2002a). p42/p44 MAPKs are intracellular targets of the CDK inhibitor purvalanol. Oncogene, 21, 6413-6424.
**11.** Zine, A., Aubert, A., Qiu,J., Therianos,S., Guillemot,F., Kageyama,R. and de Ribaupierre,F. (2001). Hes1 and Hes5 activities are required for the normal development of the hair cells in the mammalian inner ear. J.Neurosci., 21, 4712-4720.
**12.**Vesely,J., Havlicek,L., Strnad,M., Blow,J.J., Donella-Deana,A., Pinna,L., Letham,D.S., Kato,J., Detivaud,L., Leclerc,S. and Meijer,L. (1994). Inhibition of cyclin-dependent kinases by purine analogues. Eur.J.Biochem., 224, 771-786.
**13.** Hoessel,R., Leclerc,S., Endicott,J.A., Nobel,M.E., Lawrie,A., Tunnah,P., Leost,M., Damiens,E., Marie,D., Marko,D., Niederberger,E., Tang,W., Eisenbrand,G. and Meijer,L. (1999). Indirubin, the active constituent of a Chinese antileukaemia medicine, inhibits cyclin-dependent kinases. Nat.Cell Biol., 1, 60-67.
**14.**Schultz,C., Link,A., Leost,M., Zaharevitz,D.W., Gussio,R., Sausville,E.A., Meijer,L. and Kunick,C. (1999). Paullones, a series of cyclin-dependent kinase inhibitors: synthesis, evaluation of CDK1/cyclin B inhibition, and in vitro antitumor activity. J.Med.Chem., 42, 2909-2919.
**15.** Soni,R., Muller,L., Furet,P., Schoepfer,J., Stephan,C., Zumstein,M., Fretz,H. and Chaudhuri,B. (2000). Inhibition of cyclin-dependent kinase 4 (Cdk4) by fascaplysin, a marine natural product. Biochem Biophys Res Commun, 275, 877-884.
**16.** Pedreira,M.E., Dimant,B. and Maldonado,H. (1996). Inhibitors of protein and RNA synthesis block context memory and long-term habituation in the crab Chasmagnathus. Pharmacol Biochem Behav, 54, 611-617.
**17.** Liu,J., Hebert,M.D., Ye,Y., Templeton,D.J., Kung,H. and Matera,A.G. (2000). Cell cycle-dependent localization of the CDK2-cyclin E complex in Cajal (coiled) bodies. J Cell Sci, 113 (Pt 9), 1543-1552.
**18.** Favata,M.F., Horiuchi,K.Y., Manos,E.J., Daulerio,A.J., Stradley,D.A., Feeser,W.S., Van,D., Pitts,W.J., Earl,R.A., Hobbs,F., Copeland,R.A., Magolda,RL., Scherle,P.A. and Trzaskos,J.M. (1998). Identification of a novel inhibitor of mitogen-activated protein kinase kinase. J Biol Chem, 273,18623-18632.
**19.** Buolamwini,J.K. (2000). Cell cycle molecular targets in novel anticancer drug discovery. Curr.Pharm.Des, 6, 379-392**.**
**20.**Knockaert,M., Greengard,P. and Meijer,L. (2002b). Pharmacological inhibitors of cyclin-dependent kinases. Trends Pharmacol.Sci., 23, 417-425.

## Claims

1. Use of at least one kinase inhibitor selected from the group comprising purine derivatives for making drugs for inducing differentiation of supernumerary hair cells and Deiters'cells in the developing organ of Corti for treating deafness.

2. The use of claim 1, wherein said purine derivatives are selected from the group comprising roscovitine, indirubins and purvulanols.

3. The use of claim 1 or 2, wherein said kinase inhibitors are administered parenterally, rectally, topically, transdermally or orally.

4. The use of claim 3, wherein said kinase inhibitors are administered by the oral or injectable route.

5. The use of claim 4, wherein said kinase inhibitors are under the form of lozenges, compressed tablets, pills, tablets, capsules, drops, syrups, suspensions or emulsions.

6. The use of anyone of claims 3 to 5, wherein the pharmaceutical compositions comprise 100 to 1000 mg of active principle per dose unit, preferably 300 to 600 mg.

7. The use of claim 4, wherein said kinase inhibitors are administered under the form of injectable solutions for the intravenous, subcutaneous or intramuscular route, formulated from sterile or sterilizable solutions, or under the form of suspensions or emulsions.

8. The use of claim 7, wherein said injectable forms comprise 100 to 1000 mg of said compound, or a pharmaceutically acceptable salt thereof, preferably 300 to 600 mg, per dose unit.

## Patentansprüche

1. Verwendung wenigstens eines Kinase-Inhibitors, der aus der Purinderivate umfassenden Gruppe ausgewählt ist, zur Herstellung von Medikamenten zur Induzierung einer Differenzierung von überzähligen Haarzellen und Deitersschen Zellen im sich entwickelnden Cortischen Organ zur Behandlung von Taubheit.

2. Verwendung nach Anspruch 1, wobei die Purinderivate aus der Roscovitin, Indirubine und Purvulanole umfassenden Gruppe ausgewählt sind.

3. Verwendung nach Anspruch 1 oder 2, wobei die Kinase-Inhibitoren parenteral, rektal, topisch, transdermal oder oral verabreicht werden.

4. Verwendung nach Anspruch 3, wobei die Kinase-Inhibitoren über die orale oder die Injektionsroute verabreicht werden.

5. Verwendung nach Anspruch 4, wobei die Kinase-Inhibitoren in Form von Pastillen, komprimierten Tabletten, Pillen, Tabletten, Kapseln, Tropfen, Sirupen, Suspensionen oder Emulsionen vorliegen.

6. Verwendung nach einem der Ansprüche 3 bis 5, wobei die pharmazeutischen Zusammensetzungen 100 bis 1000 mg des Wirkstoffs pro Dosiseinheit, vorzugsweise 300 bis 600 mg umfassen.

7. Verwendung nach Anspruch 4, wobei die Kinase-Inhibitoren in Form von injizierbaren Lösungen für die intravenöse, subkutane oder intramuskuläre Route, die aus sterilen oder sterilisierbaren Lösungen formuliert sind, oder in Form von Suspensionen oder Emulsionen verabreicht werden.

8. Verwendung nach Anspruch 7, wobei die injizierbaren Formen 100 bis 1000 mg, vorzugsweise 300 bis 600 mg der Verbindung oder eines pharmazeutisch annehmbaren Salzes davon pro Dosiseinheit umfassen.

## Revendications

1. Utilisation d'au moins un inhibiteur de kinase, choisi dans le groupe comprenant des dérivés de purine, pour la fabrication de médicaments destinés à induire une différenciation de cellules ciliées et de cellules de Deiters surnuméraires dans l'organe de Corti en cours de développement en vue de traiter la surdité.

2. Utilisation selon la revendication 1, dans laquelle lesdits dérivés de purine sont choisis dans le groupe constitué par la roscovitine, les indirubines et les purvulanols.

3. Utilisation selon la revendication 1 ou 2, dans laquelle lesdits inhibiteurs de kinase sont administrés par voie parentérale, rectale, topique, transdermique ou orale.

4. Utilisation selon la revendication 3, dans laquelle lesdits inhibiteurs de kinase sont administrés par voie orale ou par injection.

5. Utilisation selon la revendication 4, dans laquelle lesdits inhibiteurs sont sous la forme de pastilles, de tablettes comprimées, de pilules, de comprimés, de capsules, de gouttes, de sirops, de suspensions ou d'émulsions.

6. Utilisation selon l'une quelconque des revendications 3 à 5, dans laquelle les compositions pharmaceutiques comprennent 100 à 1 000 mg de principe actif par dose unitaire, de préférence de 300 à 600 mg.

7. Utilisation selon la revendication 4, dans laquelle lesdits inhibiteurs de kinase sont administrés sous la forme de solutions injectables par voie intraveineuse, sous-cutanée ou intramusculaire, formulées à partir de solutions stériles ou stérilisables, ou sous la forme de suspensions ou d'émulsions.

8. Utilisation selon la revendication 7, dans laquelle lesdites formes injectables comprennent 100 à 1 000 mg dudit composé, ou un de ses sels acceptables sur le plan pharmaceutique, de préférence 300 à 600 mg par dose unitaire.
